(19) Europäisches Patentamt
European Patent Office
Office européen des brevets

(11) Publication number: **0 047 011**
**B1**

(12) **EUROPEAN PATENT SPECIFICATION**

(45) Date of publication of patent specification: **19.06.85**

(21) Application number: **81106827.9**

(22) Date of filing: **01.09.81**

(51) Int. Cl.$^4$: **C 07 C 59/90**, C 07 C 69/712, C 07 C 51/373, C 07 C 67/14, A 61 K 31/215

(54) **Pharmaceutical compositions containing 4-((6,7-Dihalogen-2,3-dihydro-1-oxo-1H-inden-5-yl)-oxy) butanoic acid compounds.**

(30) Priority: **02.09.80 US 183546**

(43) Date of publication of application:
**10.03.82 Bulletin 82/10**

(45) Publication of the grant of the patent:
**19.06.85 Bulletin 85/25**

(84) Designated Contracting States:
**AT BE CH DE FR GB IT LI LU NL SE**

(56) References cited:
**GB-A-1 446 953**
**GB-A-1 474 459**
**US-A-3 984 465**
**US-A-4 012 524**

(73) Proprietor: **MERCK & CO. INC.**
**126, East Lincoln Avenue P.O. Box 2000**
**Rahway New Jersey 07065 (US)**

(72) Inventor: **Cragoe, Edward J., Jr.**
**2211 Oak Terrace Drive**
**Lansdale Pennsylvania 19446 (US)**
Inventor: **Woltersdorf, Otto W., Jr.**
**200 Dorset Way**
**Chalfont Pennsylvania 19814 (US)**

(74) Representative: **Abitz, Walter, Dr.-Ing. et al**
**Abitz, Morf, Gritschneder P.O. Box 86 01 09**
**D-8000 München 86 (DE)**

**EP 0 047 011 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the invention

Traumas to the brain caused by outside physical forces acting on the skull or spinal chord (hereinafter, head or spine injury), ischemic stroke, and hydrocephalus are all characterized by edema and resultant swelling. The standard treatment has been the administration of steroids, because of their known antiinflammatory activity or procedures such as the insertion of a shunt in the case of progressive hydrocephalus. Diuretics have not been used to treat brain and spinal chord edema partly because the blood-brain barrier prevents adequate concentrations of the diuretics from reaching brain cells. Thus, any decrease in edema following diuretic administration would be a secondary or independent effect resulting from general electrolyte loss and resultant dehydration of the rest of the body. Such dehydration would be inappropriate to someone with a traumatized brain or spinal cord.

Long, *et al., Dynamics of Brain Edema*, pp. 293—300, Springer-Verlag (1976) described the use of the diuretics furosemide and acetazolamide for the treatment of certain models of brain edema in cats. Bourke, *et al., Brain Research*, 105 (1976) 309—323 described the effect of the diuretics ethacrynic acid and acetazolamide on swelling of monkey cerebrocortical slices. Nelson, *et al.* Neural Trauma, edited by A. J. Popp *et al.*, Raven Press, New York, 1979, p. 297, have described the use of ethacrynic acid in brain injury.

US—A—3 984 465 describes 4-(1-oxo-2-cyclopentyl-2-methyl-6,7-dichloro-5-indanyloxy)butyric acid (see Example 23) and its use as a medicine reducing the sodium and chloride ions in the body (see column 1, lines 23—28).

Summary of the invention

The invention comprises a pharmaceutical composition for treating gray matter edema. This edema can be the result of any of a variety of causes; for example, from external physical forces such as a blow to the head, neck or spine, a motor vehicle accident, or a fall, from ischemic stroke, from hydrocephalus, or from radiation. The pharmaceutical composition of the invention comprises a compound of the formula:

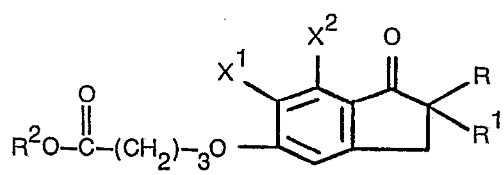

Formula I

wherein

$X^1$ and $X^2$ are halo;

R is lower alkyl of from 1 to 6 carbon atoms;

$R^1$ is hydrogen, lower alkyl of 1 to 4 carbon atoms, cycloalkyl of 3 to 6 carbon atoms, lowercycloalkylloweralkyl of 4 to 7 carbon atoms and phenyl;

$R^2$ is hydrogen or lower alkyl of 1 to 6 carbon atoms, and carboxy lower alkyl of 2 to 6 carbon atoms; and the pharmaceutically acceptable salts thereof; with the exception of 4 - [(6,7 - dichloro - 2 - cyclopentyl - 2,3 - dihydro - 2 - methyl - 1 - oxo - 1H - inden - 5 - yl)oxy]butanoic acid.

Furthermore, the invention refers to a pharmaceutical composition for treating gray matter edema comprising 4 - [(6,7 - dichloro - 2 - cyclopentyl - 2,3 - dihydro - 2 - methyl - 1 - oxo - 1H - inden - 5 - yl)oxy]butanoic acid, lactose and magnesium stearate.

Since the 2-carbon atom in the molecule is asymmetric, the compounds of the invention where R and $R^1$ are different are racemic; however, these compounds can be resolved, so that the invention includes the pure enantiometers. In addition, in some instances, the group represented by $R^2$ includes an asymmetric carbon atom. Thus, these molecules may contain two asymmetric carbon atoms and now can consist of two diastereomers, each of which consists of two enantiomers. The invention includes each diastereomer and their enantiomers whenever they exist.

A particular advantage of the compounds used for this invention is that they belong to a chemical subgroup which possess maximal anti-brain edema activity and minimal renal (diuretic) activity.

It can be seen from J. Med. Chem. *25*, 567—579 (1982) that the compounds used for this invention have an approximately just as good ability to inhibit brain tissue swelling as the (indanyloxy)acetic acid derivatives of US—A—3 984 465. At the same time, the present butyric acid derivatives have the advantage that they do not exhibit diuretic activity while the acetic acid derivatives throughout also have diuretic activity. Consequently, the present compounds are suitable for focused treatment of gray matter edema without it coming to a diuretic effect which is undesired in such cases. Reference is also made in the above-mentioned paper to the clinical advantage of nondiuretic (aryloxy)alkanoic acids.

It is possible to administer a compound of formula I concomitantly with an antiinflammatory steroid or barbiturate for the treatment of gray matter edema of the brain or spinal chord.

Detailed description of the invention

By alkyl is meant both straight- and branched-chain alkyl such as methyl, ethyl, n-butyl, sec-butyl,

iso-butyl, t-butyl, pentyl and the like. Cycloalkyl includes cyclopropyl, cyclobutyl, cyclopentyl, and cyclohexyl. By halo is meant fluoro, chloro, and bromo.

As mentioned previously, the use of the enantiomeric forms of the compound of formulae I is included within the scope of this invention. It is to be noted that one enantiomer generally possesses most or all the anti-brain edema activity expressed by the racemic compound.

Preferred compounds of formula I are those where $X^1$ and $X^2$ are both chloro, $R^1$ is cyclopentyl, and $R^2$ is H.

Other preferred compounds of formula I are those where $R^2$ is 1-carboxy-1-methylethyl.

A preferred compound is the (+) enantiomer of 4 - [(6,7 - dichloro - 2 - cyclopentyl - 2,3 - dihydro - 2 - methyl - 1 - oxo - 1H - inden - 5 - yl)oxy]butanoic acid.

Another preferred compound is 4 - [(6,7 - dichloro - 2 - cyclopentyl - 2 - ethyl - 2,3 - dihydro - 1 - oxo - 1H - inden - 5 - yl)oxy]butanoic acid.

Another preferred compound of the invention is 4 - [(6,7 - dichloro - 2 - cyclopentyl - 2,3 - dihydro - 1 - oxo - 2 - propyl - 1H - inden - 5 - yl)oxy]butanoic acid.

Another preferred compound of the invention is 4 - [(2 - butyl - 6,7 - dichloro - 2 - cyclopentyl - 2,3 - dihydro - 1 - oxo - 1H - inden - 5 - yl)oxy]butanoic acid.

Another preferred compound of the invention is the (+) enantiomer of 4 - [(2 - butyl - 6,7 - dichloro - 2 - cyclopentyl - 2,3 - dihydro - 1 - oxo - 1H - inden - 5 - yl)oxy]butanoic acid.

Another preferred compound is the (−) enantiomer of 4 - [(2 - butyl - 6,7 - dichloro - 2 - cyclopentyl - 2,3 - dihydro - 1 - oxo - 1H - inden - 5 - yl)oxy]butanoic acid.

Another preferred compound of the invention is the (+) enantiomer of 1 - carboxy - 1 - methylethyl 4 - [(6,7 - dichloro - 2 - cyclopentyl - 2,3 - dihydro - 2 - methyl - 1 - oxo - 1H - inden - 5 - yl)oxy]butanoate.

Another preferred compound of the invention is the (+) enantiomer of 1 - carboxy - 1 - methylethyl 4 - [(2 - butyl - 6,7 - dichloro - 2 - cyclopentyl - 2,3 - dihydro - 1 - oxo - 1H - inden - 5 - yl)oxy]butanoate.

Another preferred compound of the invention is the (−) enantiomer of 1 - carboxy - 1 - methylethyl 4 - [(2 - butyl - 6,7 - dichloro - 2 - cyclopentyl - 2,3 - dihydro - 1 - oxo - 1H - inden - 5 - yl)oxy]butanoate.

Included within the scope of this invention is the use of the salts, esters, and amide derivatives of formulae I compounds which are prepared by conventional methods well known to those skilled in the art, i.e., reaction of the appropriate (indanyloxy)butanoic acid and selected alcohol with dicyclohexylcarbodiimide. In addition, the ester derivatives may be prepared by the reaction of the formulae I compounds of this invention with an alcohol, for example, with a hydroxyalkanoic acid in the presence of an acid catalyst.

Esters, such as the 1-carboxy-1-methylethyl esters (II) can be prepared by heating the appropriate acylating agent (III or IV) with 2-hydroxy-isobutyric acid.

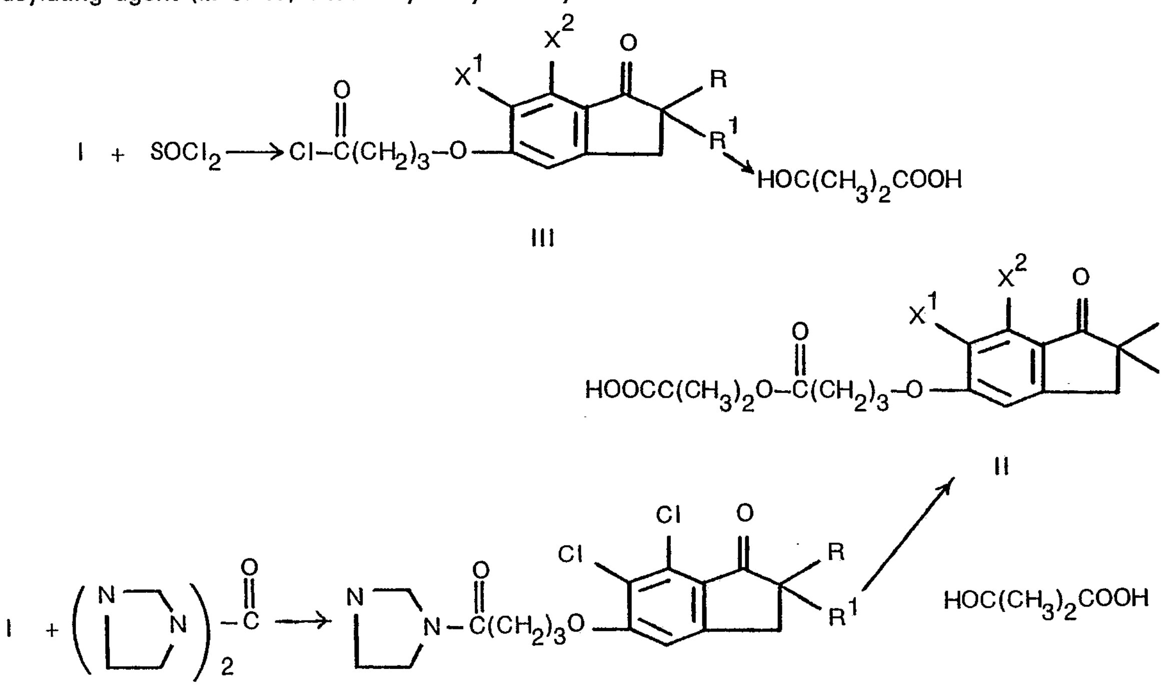


III

II

IV

The intermediate III can be made by warming I with $SOCl_2$ in an inert solvent and IV can be synthesized by treating I with 1,1′-carbonyldiimidazole in a solvent such as tetrahydrofuran.

The amide derivatives may be prepared by converting a formulae I compound to its corresponding acid chloride by treatment with thionyl chloride followed by treating said acid chloride with ammonia, an appropriate mono-lower alkyl amine, di-lower alkyl amine or a hetero amine, such as piperidine, morpholine and the like, to produce the corresponding amide compound. These and other equivalent methods for the preparation of the salts, esters, and amide derivatives of the instant products will be apparent to one having ordinary skill in the art and to the extent that said derivatives are both non-toxic and pharmacologically acceptable, said derivatives are the functional equivalent of the corresponding compounds of formulae I.

The preferred salts are: sodium, potassium, ammonium, ethanolamine, diethanolamine, triethanolamine, N-methylpiperazine, piperazine, cyclohexylamine, N-methylglucamine, N-methylglucosamine, tetramethylammonium, and the like.

Inasmuch as there is a wide variety of symptoms and severity associated with gray matter edema, particularly when it is caused by blows to the head or spinal chord, the precise treatment protocol is left to the practitioner. It is up to the practitioner to determine the patient's response to treatment and to vary the dosages accordingly. A recommended dosage range is from 1 µg to 2 mg/kg of body weight as a primary dose and a sustaining dose of half to equal the primary dose, every 12 to 24 hours.

The compositions of this invention can be administered by a variety of established methods, including intravenously, intramuscularly, subcutaneously, and orally. As with dosage, the precise mode of administration is left to the discretion of the practitioner.

Since some of the compound used for this invention have diuretic properties which may vary in intensity from compound to compound, it is recommended that the person under therapy receive an intravenous infusion equivalent to the lost water and electrolyte. Here again the rate of infusion and the solutions used are left to the discretion of the practitioner.

Studies on human pathological tissues have revealed that ischemic insult to the brain is a major concomitant of head injury.

Recent studies in experimental head injury by R. S. Bourke et al. (R. S. Bourke, M. A. Daze and H. K. Kimelberg, Monograph of the International Glial Cell Symposium, Liège, Belgium, August 29—31, 1977 (in press) and references cited therein) and experimental stroke by J. H. Garcia et al. (J. H. Garcia, H. Kalimo, Y. Kamijyo and B. F. Trump, Virchous Archiv. B (1977), (in press)) indicate that astroglial swelling, a secondary and potentially inhibitable process, is a fundamental pathophysiological response to ischemic/traumatic brain insult in both pathological disorders. Furthermore, astroglial swelling is believed to reduce oxygen available to neurons by prolongation of the oxygen diffusion pathway. Thus, the damage to cerebral grey matter may be far more extensive as a result of pathological events secondary to astroglial swelling than as a result of damage inflicted by the initial ischemic/traumatic insult. Consequently, it is of prime importance that the treatment commence as soon as possible after the initial trauma in order to minimize the brain cell damage and the possibility of death or permanent paralysis.

As previously mentioned, it is possible to administer a compound of formula I or a pharmaceutically acceptable salt, ester, or amide thereof together with antiinflammatory steroids, and/or barbiturates. These steroids are of some, albeit limited, use in control of white matter edema associated with ischemic stroke and head injury. Steroid therapy is given according to established practice as a supplement to the compound of formula I as taught elsewhere herein.

The compounds used for this invention generally are prepared by the synthetic methods described in U.S. Patent 3,984,465. The examples which follow illustrate the compounds used for this invention and the methods by which they are prepared. However, the examples are only illustrative and it will be apparent to those having ordinary skill in the art that all of the products embraced by formula I, supra., also may be prepared in an analogous manner by substituting the appropriate starting materials for those set forth in the examples.

Example 1

4-[(6,7-Dichloro-2-cyclopentyl-2,3-dihydro-2-methyl-1-oxo-1H-inden-5-yl)oxy]butanoic acid

Step A: 2',3'-Dichloro-4'-methoxy-2-cyclopentylacetophenone

2,3-Dichloroanisole (57.8 g, 0.327 mole) is dissolved in dichloromethane (300 ml.) and cyclopentylacetyl chloride (52.7 g., 0.367 mole) is added. The solution is cooled to 5°C. and aluminum chloride (48.0 g. 0.36 mole) is added gradually over a one-hour period at 5°C. The mixture is stirred for two hours at 5°C. and at 20°—25°C. for 16 hours and then poured into 1 liter of ice water containing 150 ml. of 12N hydrochloric acid. The organic phase is separated and the aqueous phase is extracted with dichloromethane. The combined organic phases are washed first with a sodium chloride solution, then a 10% sodium hydroxide solution and again with sodium chloride solution and finally dried over magnesium sulfate. On evaporation of the solvent a brown solid is obtained which is crystallized from hexane to obtain 53.2 g. of 2',3' - dichloro - 4' - methoxy - 2 - cyclopentylacetophenone, m.p. 60°—61.5°C.

Elemental analysis for $C_{15}H_{16}Cl_2O_2$:

| | |
|---|---|
| Calc: | C, 58.55; H, 5.62; |
| Found: | C, 58.72; H, 5.71. |

Step B: 4-(2-Cyclopentyl-2-methyleneacetyl)-2,3-dichloroanisole

2,3-Dichloro-4-methoxy-2-cyclopentylacetophenone (51.6 g., 0.18 mole) is dissolved in dioxane (460 ml.) and paraformaldehyde (21.6 g., 0.72 mole) and concentrated sulfuric acid (9.65 g.) are added. The mixture is heated at 80°—85°C. for 20 hours. The dioxane is evaporated at reduced pressure. Water is added to the residual gum which then is extracted into ether. The ether extract is washed with water and dried over magnesium sulfate. The ether is evaporated and upon triturating the residue with hexane (5 ml.) there is obtained a solid that is crystallized from ligroin to obtain 4 - (2 - cyclopentyl - 2 - methyleneacetyl) - 2,3 - dichloroanisole (33.3 g.), m.p. 59°—63°C. Crystallization from butyl chloride affords a sample (m.p. 66°—67.5°C) for analysis.

Elemental analysis for $C_{16}H_{16}Cl_2O_2$:
Calc.: C, 60.21; H, 5.37;
Found: C, 60.19; H, 5.42.

Step C: 6,7-Dichloro-2-cyclopentyl-2,3-dihydro-5-methoxy-1H-inden-1-one

4-(2-Cyclopentyl-2-methyleneacetyl)-2,3-dichloroanisole (33.3 g.) is dissolved in 98% sulfuric acid (150 ml.) and stirred at 20°C. for 1.5 hours. The solution then is added dropwise with stirring to ice water. The aqueous phase is decanted from the gummy product and fresh water is added. After 20 hours the gum solidifies and is crystallized from hexane-benzene (3:1) to obtain 6,7-dichloro-2-cyclopentyl-2,3-dihydro-5-methoxy-1H-inden-1-one, m.p. 116°—117°C.

Elemental analysis for $C_{16}H_{16}Cl_2O_2$:
Calc.: C, 60.21; H, 5.37;
Found: C, 60.29; H, 5.35.

Step D: 6,7-Dichloro-2-cyclopentyl-2,3-dihydro-5-methoxy-2-methyl-1H-inden-1-one

6,7-Dichloro-2-cyclopentyl-2,3-dihydro-5-methoxy-1H-inden-1-one (7.5 g., 0.025 mole) is dissolved in dry 1,2-dimethoxyethane (200 ml.) under nitrogen. Sodium hydride (57% in mineral oil; 1.16 g., 0.0275 mole) is then added and the mixture is stirred at 80° until evolution of hydrogen ceases (2 hours). The solution is cooled and methyl iodide (7.5 ml.) is added, the mixture is again brought to reflux and then cooled. Most of the 1,2-dimethoxyethane is evaporated and water is added to the residue which soon solidifies and is crystallized from methylcyclohexane and from ethanol-water (4:1) to obtain 3.4 g. of 6,7-dichloro-2-cyclopentyl-2,3-dihydro-5-methoxy-2-methyl-1H-inden-1-one, m.p. 109°—111.5°C.

Elemental analysis for $C_{17}H_{18}Cl_2O_2$:
Calc.: C, 61.35; H, 5.79;
Found: C, 61.71; H, 5.84.

Step E: 6,7-Dichloro-2-cyclopentyl-2,3-dihydro-5-hydroxy-2-methyl-1H-inden-1-one

6,7-Dichloro-2-cyclopentyl-2,3-dihydro-5-methoxy-2-methyl-1H-inden-1-one (3.4 g., 0.0109 mole) is added to dry heptane (180 ml.) and aluminum chloride (4.36 g., 0.0327 mole) is added. The mixture is refluxed for one hour and the heptane is decanted from the gummy residue which then is added to ice water (200 ml.) containing 12N hydrochloric acid (15 ml.). The solid that separates is crystallized from benzene to obtain 2.77 g. of 6,7 - dichloro - 2 - cyclopentyl - 2,3 - dihydro - 5 - hydroxy - 2 - methyl - 1H - inden - 1 - one, m.p. 190°—194°C.

Elemental analysis for $C_{16}H_{16}Cl_2O_2$:
Calc.: C, 60.21; H, 5.37;
Found: C, 60.43; H, 5.41.

Step F: 4-[(6,7-Dichloro-2-cyclopentyl-2,3-dihydro-2-methyl-1-oxo-1H-indene-5-yl)oxy]butanoic acid

A stirred mixture of 6,7-dichloro-2-cyclopentyl-2,3-dihydro-5-hydroxy-2-methyl-1H-inden-1-one (30.9 g., 0.1 mole), potassium carbonate (15.2 g., 0.11 mole) and ethyl 4-bromobutyrate (23.5 g., 0.11 mole) in DMF (200 ml.) is heated at 55—60°C for 2 hours, then treated with water (200 ml.) and 10N sodium hydroxide (40 ml.) and heated at 95°C for 2 hours. The reaction mixture is poured into ice water (500 ml.) containing conc. hydrochloric acid (50 ml.) extracted with ether which is washed with brine and dried over $MgSO_4$. The ether is evaporated at reduced pressure and the resultant oil crystallized from butyl chloride to give 16 g. of 4 - [(6,7 - dichloro - 2 - cyclopentyl - 2,3 - dihydro - 2 - methyl - 1 - oxo - 1H - inden - 5 - yl)oxy]butanoic acid which melts at 154°C.

Analysis for $C_{19}H_{22}Cl_2O_4$:
Calc.: C, 59.23; H, 5.76;
Found: C, 59.25; H, 5.82.

Example 2

4-[(6,7-Dichloro-2-cyclopentyl-2-ethyl-2,3-dihydro-1-oxo-1H-inden-5-yl)oxy]butanoic acid

Step A: 6,7-Dichloro-2-cyclopentyl-2-ethyl-2,3-dihydro-5-methoxy-1H-inden-1-one

By following substantially the procedure described in Example 1, Step D, but substituting for the methyl iodide therein described an equimolar quantity of ethyl iodide and substituting a mixture of equal volumes of dimethylformamide and toluene for the 1,2-dimethoxyethane used therein, there is obtained

6,7 - Dichloro - 2 - cyclopentyl - 2 - ethyl - 2,3 - dihydro - 5 - methoxy - 1H - inden - 1 - one, which melts at 163°C.

Analysis for $C_{17}H_{20}Cl_2O_2$

| | |
|---|---|
| Calc.: | C, 62.39; H, 6.16; |
| Found: | C, 62.35; H, 6.36. |

Step B: 6,7-Dichloro-2-cyclopentyl-2-ethyl-2,3-dihydro-5-hydroxy-1H-inden-1-one

By following substantially the procedure described in Example 8, Step A, but substituting for the (+)[(6,7 - dichloro - 2 - cyclopentyl - 2,3 - dihydro - 2 - methyl - 1 - oxo - 1H - inden - 5 - yl)oxy]acetic acid therein described, an equimolar quantity of 6,7 - dichloro - 2 - cyclopentyl - 2,3 - dihydro - 2 - ethyl - 5 - methoxy - 1H - inden - 1 - one there is obtained 6,7 - dichloro - 2 - cyclopentyl - 2 - ethyl - 2,3 - dihydro - 5 - hydroxy - 1H - inden - 1 - one which is used in Step C without further purification.

Step C: 4-[(6,7-Dichloro-2-cyclopentyl-2-ethyl-2,3-dihydro-1-oxo-1H-inden-5-yl)oxy]butanoic acid

By following substantially the procedure described in Example 1, Step F, but substituting for the 6,7 - dichloro - 2 - cyclopentyl - 2,3 - dihydro - 5 - hydroxy - 2 - methyl - 1H - inden - 1 - one an equimolar quantity of 6,7 - dichloro - 2 - cyclopentyl - 2 - ethyl - 2,3 - dihydro - 5 - hydroxy - 1H - inden - 1 - one therein described, there is obtained 4 - [(6,7 - dichloro - 2 - cyclopentyl - 2 - ethyl - 2,3 - dihydro - 1 - oxo - 1H - inden - 5 - yl)oxy]butanoic acid which melts at 189°—193°C.

Analysis for $C_{20}H_{24}Cl_2O_4$

| | |
|---|---|
| Calc.: | C, 60.16; H, 6.06; |
| Found: | C, 59.91; H, 6.13. |

Example 3

4-[(6,7-Dichloro-2-cyclopentyl-2,3-dihydro-1-oxo-2-propyl-1H-inden-5-yl)oxy]butanoic acid

Step A: 6,7-Dichloro-2-cyclopentyl-2,3-dihydro-2-propyl-5-methoxy-1H-inden-1-one

By following substantially the procedure described in Example 2, Step A, but substituting for the ethyl iodide therein described, an equimolar quantity of propyl iodide there is obtained 6,7 - dichloro - 2 - cyclopentyl - 2,3 - dihydro - 2 - propyl - 5 - methoxy - 1H - inden - 1 - one.

Step B: 6,7-Dichloro-2-cyclopentyl-2,3-dihydro-5-hydroxy-2-propyl-1H-inden-1-one

By following substantially the procedure described in Example 2, Step B, but substituting for the 6,7 - dichloro - 2 - cyclopentyl - 2,3 - dihydro - 5 - methoxy - 2 - ethyl - 1H - inden - 1 - one therein described, an equimolar quantity of 6,7 - dichloro - 2 - cyclopentyl - 2,3 - dihydro - 5 - methoxy - 2 - propyl - 1H - inden - 1 - one there is obtained 6,7 - dichloro - 2 - cyclopentyl - 2,3 - dihydro - 5 - hydroxy - 2 - propyl - 1H - inden - 1 - one, which is used in Step C without further purification.

Step C: 4-[(6,7-dichloro-2-cyclopentyl-1-2,3-dihydro-oxo-2-propyl-1H-inden-5-yl)oxy]butanoic acid

By following substantially the procedure described in Example 2, Step C, but substituting for the 6,7 - dichloro - 2 - cyclopentyl - 2,3 - dihydro - 5 - hydroxy - 2 - ethyl - 1H - inden - 1 - one, an equimolar quantity of 6,7 - dichloro - 2 - cyclopentyl - 2,3 - dihydro - 5 - hydroxy - 2 - propyl - 1H - inden - 1 - one therein described, there is obtained 4-[(6,7 - dichloro - 2 - cyclopentyl - 2,3-dihydro - 1 - oxo - 2 - propyl - 1H - inden - 5 - yl)oxy]butanoic acid, which melts at 140°—141°C.

Analysis for $C_{21}H_{26}Cl_2O_4$

| | |
|---|---|
| Calc.: | C, 61.02; H, 6.34; |
| Found: | C, 61.05; H, 6.53. |

Example 4

4-[(2-Butyl-6,7-dichloro-2-cyclopentyl-2,3-dihydro-1-oxo-1H-inden-5-yl)oxy]butanoic acid

Step A: 2-Butyl-6,7-dichloro-2-cyclopentyl-2,3-dihydro-5-methoxy-1H-inden-1-one

By following substantially the procedure described in Example 2, Step A, but substituting for the ethyl iodide therein described, an equimolar quantity of butyl iodide, there is obtained 2 - butyl - 6,7 - dichloro - 2 - cyclopentyl - 2,3 - dihydro - 5 - methoxy - 1H - inden - 1 - one.

Step B: 2-Butyl-6,7-dichloro-2-cyclopentyl-2,3-dihydro-5-hydroxy-1H-inden-1-one

By following substantially the procedure described in Example 2, Step B, but substituting for the 6,7 - dichloro - 2 - cyclopentyl - 2 - ethyl - 2,3 - dihydro - 5 - methoxy - 1H - inden - 1 - one therein described, an equimolar quantity of 2 - butyl - 6,7 - dichloro - 2 - cyclopentyl - 2,3 - dihydro - 5 - methoxy - 1H - inden - 1 - one there is obtained 2 - butyl - 6,7 - dichloro - 2 - cyclopentyl - 2,3 - dihydro - 5 - hydroxy - 1H - inden - 1 - one, which is used in Step C without further purification.

Step C: 4-[(2-Butyl-6,7-dichloro-2-cyclopentyl-2,3-dihydro-1-oxo-1H-inden-5-yl)oxy]butanoic acid

By following substantially the procedure described in Example 2, Step C, but substituting for the 6,7 - dichloro - 2 - cyclopentyl - 2 - ethyl - 2,3 - dihydro - 5 - hydroxy - 1H - inden - 1 - one, an equimolar quantity of 2 - butyl - 6,7 - dichloro - 2 - cyclopentyl - 2,3 - dihydro - 5 - hydroxy - 1H - inden - 1 - one,

therein described, there is obtained 4 - [(2 - butyl - 6,7 - dichloro - 2 - cyclopentyl - 2,3 - dihydro - 1 - oxo - 1H - inden - 5 - yl)oxy] - butanoic acid, which melts at 156.5°—159°C.

Analysis for $C_{22}H_{28}Cl_2O_4$

Calc.: C, 61.83; H, 6.61;

Found: C, 61.64; H, 6.73.

Example 5

4-[(6,7-Dichloro-2-cyclopentyl-2,3-dihydro-1-oxo-2-pentyl-1H-inden-5-yl)oxy]butanoic acid

Step A: 6,7-Dichloro-2-cyclopentyl-2,3-dihydro-5-methoxy-2-pentyl-1H-inden-1-one

By following substantially the procedure described in Example 2, Step A, but substituting for the ethyl iodide therein described, an equimolar quantity of pentyl iodide, there is obtained 6,7 - dichloro - 2 - cyclopentyl - 2,3 - dihydro - 5 - methoxy - 2 - pentyl - 1H - inden - 1 - one, which is used in Step B without further purification.

Step B: 6,7-Dichloro-2-cyclopentyl-2,3-dihydro-5-hydroxy-2-pentyl-1H-inden-1-one

By following substantially the procedure described in Example 2, Step B, but substituting for the 6,7 - dichloro - 2 - cyclopentyl - 2,3 - dihydro - 5 - methoxy - 2 - ethyl - 1H - inden - 1 - one - therein described, an equimolar quantity of 6,7 - dichloro - 2 - cyclopentyl - 2,3 - dihydro - 5 - methoxy - 2 - pentyl - 1H - inden - 1 - one there is obtained 6,7 - dichloro - 2 - cyclopentyl - 2,3 - dihydro - 5 - hydroxy - 2 - pentyl - 1H - inden - 1 - one which is used in Step C without further purification.

Step C: 4-[(6,7-Dichloro-2-cyclopentyl-2,3-dihydro-1-oxo-2-pentyl-1H-inden-5-yl)oxy]butanoic acid

By following substantially the procedure described in Example 2, Step C, but substituting for the 6,7 - dichloro - 2 - cyclopentyl - 2 - ethyl - 2,3 - dihydro - 5 - hydroxy - 1H - inden - 1 - one an equimolar quantity of 6,7 - dichloro - 2 - cyclopentyl - 2,3 - dihydro - 5 - hydroxy - 2 - pentyl - 1H - inden - 1 - one therein described, there is obtained 4 - [(6,7 - dichloro - 2 - cyclopentyl - 2,3 - dihydro - 1 - oxo - 2 - pentyl - 1H - inden - 5 - yl)oxy]butanoic acid.

Example 6

4-[(6,7-Dichloro-2-cyclopentyl-2-hexyl-2,3-dihydro-1-oxo-1H-inden-5-yl)oxy]butanoic acid

Step A: 6,7-Dichloro-2-cyclopentyl-2,3-dihydro-2-hexyl-5-methoxy-1H-inden-1-one

By following substantially the procedure described in Example 2, Step A, but substituting for the ethyl iodide therein described, an equimolar quantity of hexyl iodide there is obtained 6,7 - dichloro - 2 - cyclopentyl - 2 - hexyl - 2,3 - dihydro - 5 - methoxy - 1H - inden - 1 - one.

Step B: 6,7-Dichloro-2-cyclopentyl-2-hexyl-2,3-dihydro-5-hydroxy-1H-inden-1-one

By following substantially the procedure described in Example 2, Step B, but substituting for the 6,7 - dichloro - 2 - cyclopentyl - 2 - ethyl - 2,3 - dihydro - 5 - methoxy - 1H - inden - 1 - one therein described, an equimolar quantity of 6,7 - dichloro - 2 - cyclopentyl - 2 - hexyl - 2,3 - dihydro - 5 - methoxy - 1H - inden - 1 - one there is obtained 6,7 - dichloro - 2 - cyclopentyl - 2 - hexyl - 2,3 - dihydro - 5 - hydroxy - 1H - inden - 1 - one, which is used in Step C without further purification.

Step C: 4-[(6,7-dichloro-2-cyclopentyl-2-hexyl-2,3-dihydro-1-oxo-1H-inden-5-yl)oxy]butanoic acid

By following substantially the procedure described in Example 2, Shep C, but substituting for the 6,7 - dichloro - 2 - cyclopentyl - 2 - ethyl - 2,3 - dihydro - 5 - hydroxy - 1H - inden - 1 - one, an equimolar quantity of 6,7 - dichloro - 2 - cyclopentyl - 2 - hexyl - 2,3 - dihydro - 5 - hydroxy - 1H - inden - 1 - one therein described, there is obtained 4 - [(6,7 - dichloro - 2 - cyclopentyl - 2 - hexyl - 2,3 - dihydro - 1 - oxo - 1H - inden - 5 - yl)oxy]butanoic acid.

Example 7

Ethyl 4-[(6,7-dichloro-2-cyclopentyl-2,3-dihydro-2-methyl-1-oxo-1H-inden-5-yl)oxy]butanoate

To a suspension of 4-[(6,7-dichloro-2-cyclopentyl-2,3-dihydro-2-methyl-1-oxo-1H-inden-5-yl)oxy]butanoic acid (0.5 g. 0.0012 mole) in absolute ethanol (50 ml) is added conc. sulfuric acid and the mixture is refluxed for 1 hr., treated with 3Å molecular sieves and stirred at 25° for 18 hours. The reaction mixture is treated with more conc. sulfuric acid (0.2 ml) refluxed for 1 hr, filtered and treated with solid potassium carbonate. The ethanol is distilled at reduced pressure to give ethyl 4 - [(6,7 - dichloro - 2 - cyclopentyl - 2,3 - dihydro - 2 - methyl - 1 - oxo - 1H - inden - 5 - yl)oxy]butanoate which is purified by chromatography on silica gel eluted with methylene chloride-tetrahydrofuran (100:1).

Analysis for $C_{21}H_{26}Cl_2O_4$

Calc.: C, 61.02; H, 6.34;

Found: C, 60.93; H, 6.39.

The product also can be prepared by mixing 4 - [(6,7 - dichloro - 2 - cyclopentyl - 2,3 - dihydro - 2 - methyl - 1 - oxo - 1H - inden - 5 - yl)oxy]butanoic acid (0.5 g, 0.0012 mole) with ethanol (0.0024 mole) in methylene chloride and adding dicyclohexylcarbodiimide (0.26 g, 0.0013 mole), filtering and purifying by chromatography as described above.

Example 8

(+) Sodium 4-[(6,7-dichloro-2-cyclopentyl-2,3-dihydro-2-methyl-1-oxo-1H-inden-5-yl)oxy]butanoate hemisemihydrate

Step A: (+) 6,7-Dichloro-2-cyclopentyl-2,3-dihydro-5-hydroxy-2-methyl-1H-inden-1-one

A 19.5 g. sample of (+) [(6,7-dichloro-2-cyclopentyl-2,3-dihydro-2-methyl-1-oxo-1H-inden-5-yl)oxy]acetic acid is added to stirred molten (190°C) pyridine hydrochloride, stirred for 1 hour then poured into ice water (800 ml). The (+) 6,7-dichloro-2-cyclopentyl-2,3-dihydro-5-hydroxy-2-methyl-1H-inden-1-one, which separates is filtered, rinsed with water and dried.

Step B: (+) Sodium 4-[(6,7-dichloro-2-cyclopentyl-2,3-dihydro-2-methyl-1-oxo-1H-inden-5-yl)oxy]butanoate hemisemihydrate

A mixture of (+) 6,7-dichloro-2-cyclopentyl-2,3-dihydro-5-hydroxy-2-methyl-1H-inden-1-one (14.25 g, 0.046 mole) potassium carbonate (12.5 g, 0.09 mole) and ethyl 4-bromobutanoate (17.5 g, 0.08 mole) in DMF (150 ml) is stirred at 55°C for 4 hours then poured into ice water and extracted with ether, washed with water and the ether evaporated at reduced pressure. The residual oil is dissolved in acetic acid (150 ml) and 5% hydrochloric acid (50 ml), heated at 95°C for 2 1/2 hours, cooled and treated with i.e. (50 g) to give 17.65 g of (+) [(6,7 - dichloro - 2 - cyclopentyl - 2,3 - dihydro - 2 - methyl - 1 - oxo - 1H - inden - 5 - yl)oxy]butanoic acid which melts at 75°—77°C.

$$[\alpha]_D^{25} \text{ (C=, acetone)} = +36.8°.$$

The butanoic acid is dissolved in water 800 ml) containing a slight excess of N-methylpiperazine, filtered and treated with ice and slight excess of 10N sodium hydroxide. The (+) sodium 4 - [(6,7 - dichloro - 2 - cyclopentyl - 2,3 - dihydro - 2 - methyl - 1 - oxo - 1H - inden - 5 - yl)oxy]butanoate hemisemihydrate, which separates melts at 204°—205°C after recrystallization from water.

Analysis for $C_{19}H_{21}Cl_2NaO_4(1/4\ H_2O)$

| | |
|---|---|
| Calc.: | C, 55.41; H, 5.26; Cl, 17.22; |
| Found: | C, 55.01; H, 5.26; Cl, 17.50. |

Example 9

(+) 4-[(2-Butyl-6,7-dichloro-2-cyclopentyl-2,3-dihydro-1-oxo-1H-inden-5-yl)oxy]butanoic acid

Step A: (+) [(2-Butyl-6,7-dichloro-2-cyclopentyl-2,3-dihydro-1-oxo-1H-inden-5-yl)oxy]acetic acid

Equimolar amounts of racemic [(2 - butyl - 6,7 - dichloro - 2 - cyclopentyl - 2,3 - dihydro - 1 - oxo - 1H - inden - 5 - yl)oxy]acetic acid (26.1 g, 0.065 mole) and (+) cinchonine (19.2 g, 0.065 mole) are dissolved in dimethylformamide (400 ml) at 110°C, then allowed to cool to ambient temperature. The salt which precipitates (27 g) is purified by ten crystallizations from dimethylformamide. This diastereomeric salt (10.7 g) is partitioned between water and ether and acidified with 6N HCl to liberate the other soluble free acid. The ether extracts are washed with dilute aqueous HCl followed by water and then dried over anhydrous magnesium sulfate. After filtration and concentration *in vacuo*, there is obtained 5.6 g of (+) [(2 - butyl - 6,7 - dichloro - 2 - cyclopentyl - 2,3 - dihydro - 1 - oxo - 1H - inden - 5 - yl)oxy]acetic acid which melts at 173—174°C, $[\alpha]_D^{24}+19.1°$ (C=5, ethanol).

Step B: (+) 2-Butyl-6,7-dichloro-2-cyclopentyl-2,3-dihydro-5-hydroxy-1H-inden-1-one

By following substantially the procedure in Example 8, Step A, but substituting for (+) [(6,7 - dichloro - 2 - cyclopentyl - 2,3 - dihydro - 2 - methyl - 1 - oxo - 1H - inden - 5 - yl)oxy]acetic acid an equimolar quantity of (+) [(2 - butyl - 6,7 - dichloro - 2 - cyclopentyl - 2,3 - dihydro - 1 - oxo - 1H - inden - 5 - yl)oxy]acetic acid, there is obtained (+) 2 - butyl - 6,7 - dichloro - 2 - cyclopentyl - 2,3 - dihydro - 5 - hydroxy - 1H - inden - 1 - one which is used in Step C without further purification.

Step C: (+) 4-[(2-Butyl-6,7-dichloro-2-cyclopentyl-2,3-dihydro-1-oxo-1H-inden-5-yl)oxy]butanoic acid

By following substantially the procedure in Example 8, Step B, but substituting for (+) 6,7 - dichloro - 2 - cyclopentyl - 2,3 - dihydro - 5 - hydroxy - 2 - methyl - 1H - inden - 1 - one an equimolar quantity of (+) 2 - butyl - 6,7 - 2 - cyclopentyl - 2,3 - dihydro - 5 - hydroxy - 1H - inden - 1 - one described therein, there is obtained (+) 4 - [(2 - butyl - 6,7 - dichloro - 2 - cyclopentyl - 2,3 - dihydro - 1 - oxo - 1H - inden - 5 - yl)oxy]butanoic acid which melts at 139—139.5°C, $[\alpha]_D^{25}+18.4°$ (C=, ethanol).

Analysis for $C_{22}H_{28}Cl_2O_4$:

| | |
|---|---|
| Calc.: | C, 61.83; H, 6.60; Cl, 16.59; |
| Found: | C, 61.96; H, 6.78; Cl, 16.61. |

Example 10

(−) 4-[(2-Butyl-6,7-dichloro-2-cyclopentyl-2,3-dihydro-1-oxo-1H-inden-5-yl)oxy]butanoic acid

Step A: (−) [(2-Butyl-6,7-dichloro-2-cyclopentyl-2,3-dihydro-1-oxo-1H-inden-5-yl)oxy]acetic acid

The initial filtrate from Example 9, Step A, which is enriched with the (−)enantiomer and is in the form of the cinchonine salt is partitioned between water and ether, is acidified with 6N hydrochloric acid to liberate the ether soluble free acid. By following substantially the procedure in Example 9, Step A, but

substituting (−) cinchonidine for (+) cinchonine there is obtained (−) [(2 - butyl - 6,7 - dichloro - 2 - cyclopentyl - 2,3 - dihydro - 1 - oxo - 1H - inden - 5 - yl)oxy]acetic acid which melts at 172—173.5°C, $[\alpha]_D^{24}-18.6°$ (C=5, ethanol).

Step B: (−) 2-Butyl-6,7-dichloro-2-cyclopentyl-2,3-dihydro-5-hydroxy-1H-inden-1-one

By following substantially the procedure in Example 8, Step A, but substituting for (+) 6,7 - dichloro - 2 - cyclopentyl - 2,3 - dihydro - 2 - methyl - 1 - oxo - 1H - inden - 5 - yl)oxy]acetic acid an equimolar quantity of (−) [(2 - butyl - 6,7 - dichloro - 2 - cyclopentyl - 2,3 - dihydro - 1 - oxo - 1H - inden - 5 - yl)oxy]acetic acid, there is obtained (−) 2 - butyl - 6,7 - dichloro - 2 - cyclopentyl - 2,3 - dihydro - 5 - hydroxy - 1H - inden - 1 - one which is used in Step C without further purification.

Step C: (−) 4-[(2-Butyl-6,7-dichloro-2-cyclopentyl-2,3-dihydro-1-oxo-1H-inden-5-yl)oxy]butanoic acid

By following substantially the procedure in Example 8, Step B, but substituting for (+) 6,7 - dichloro - 2 - cyclopentyl - 2,3 - dihydro - 5 - hydroxy - 2 - methyl - 1H - inden - 1 - one, an equimolar quantity of (−) 2 - butyl - 6,7 - dichloro - 2 - cyclopentyl - 2,3 - dihydro - 5 - hydroxy - 1H - inden - 1 - one therein described, there is obtained (−) 4 - [(2 - butyl - 6,7 - dichloro - 2 - cyclopentyl - 2,3 - dihydro - 1 - oxo - 1H - inden - 5 - yl)oxy]butanoic acid which melts at 139—139.5°C, $[\alpha]_D^{25}-17.7°$ (C=5, ethanol).

Analysis for $C_{22}H_{28}Cl_2O_4$

| | |
|---|---|
| Calc.: | C, 61.83; H, 6.60; Cl, 16.59; |
| Found: | C, 61.92; H, 6.83; Cl, 16.44. |

Example 11

1-Carboxy-1-methylethyl (+) 4-[6,7-dichloro-2-cyclopentyl-2,3-dihydro-2-methyl-1-oxo-1H-inden-5-yl)oxy]-butanoate

Step A: 1-{(+)-4-[6,7-dichloro-2-cyclopentyl-2,3-dihydro-2-methyl-1-oxo-1H-inden-5-yl)oxy]butanoyl}-imidazole

(+)-4-[(6,7-Dichloro-2-cyclopentyl-2,3-dihydro-2-methyl-1-oxo-1H-inden-5-yl)oxy]butanoic acid (Example 8, Step B) (0.77 g, 0.002 mole) is suspended in dry tetrahydrofuran (20 ml) and cooled to 0°C. A solution of 1,1′-carbonyldiimidazole (0.32 g, 0.002 mole) in tetrahydrofuran (5 ml) is added. The solution of 1 - {(+) - 4 - [(6,7 - dichloro - 2 - cyclopentyl - 2,3 - dihydro - 2 - methyl - 1 - oxo - 1H - inden - 5 - yl)oxy]butanoyl}imidazole that is formed is used in the next step without isolation.

Step B: 1-Carboxy-1-methylethyl (+) 4-[(6,7-dichloro-2-cyclopentyl-2,3-dihydro-2-methyl-1-oxo-1H-inden-5-yl)oxy]butanoate

To the solution of 1-{(+) 4-[(6,7-dichloro-2-cyclopentyl-2,3-dihydro-2-methyl-1-oxo-1H-inden-5-yl)oxy]butanoyl}imidazole (0.002 mole) prepared in Step A at 0°C is added with stirring 2-hydroxyisobutyric acid (0.21 g., 0.002 mole) and a catalytic amount of sodium hydride (10 mg). After stirring overnight at the ambient temperature, the colorless reaction mixture is concentrated *in vacuo* at 50°C. the resultant yellow liquid is chromatographed using a silica-gel (60 gm) column and eluted with a mixture of methylene chloride, tetrahydrofuran and acetic acid (100/2/1 v.v.v) to give analytically pure 1 - carboxy - 1 - methylethyl (+) 4 - [(6,7 - dichloro - 2 - cyclopentyl - 2,3 - dihydro - 2 - methyl - 1 - oxo - 1H - inden - 5 - yl)oxy]butanoate.

Example 12

1-Carboxy-1-methylethyl (+)-4-[(2-butyl-6,7-dichloro-2-cyclopentyl-2,3-dihydro-1-oxo-1H-inden-5-yl)oxy]-butanoate

Step A; 1-{(+) 4-[(2-butyl-6,7-dichloro-2-cyclopentyl-2,3-dihydro-1-oxo-1H-inden-5-yl)oxy]butanoyl}-imidazole

By substituting an equimolar amount of (+) 4 - [2 - butyl - 6,7 - dichloro - 2 - cyclopentyl - 2,3 - dihydro - 1 - oxo - 1H - inden - 5 - yl)oxy]butanoic acid for the (+) 4 - [(6,7 - dichloro - 2 - cyclopentyl - 2,3 - dihydro - 2 - methyl - 1 - oxo - 1H - inden - 5 - yl)oxy]butanoic acid used in Example 11A and conducting the reaction as described in Example 11A, there is obtained 1-{(+) 4 - [(2 - butyl - 6,7 - dichloro - 2 - cyclopentyl - 2,3 - dihydro - 1 - oxo - 1H - inden - 5 - yl)oxy]butanoyl}imidazole.

Step B: 1-Carboxy-1-methylethyl (+) 4-[(2-butyl-6,7-dichloro-2-cyclopentyl-2,3-dihydro-1-oxo-1H-inden-5-yl)oxy]butanoate

By substituting an equimolar amount of 1 - (+) {4-[(2 - butyl - 6,7 - dichloro - 2 - cyclopentyl - 2,3 - dihydro - 1 - oxo - 1H - inden - 5 - yl)oxy]butanoyl}imidazole for the 1 - {(+) 4 - [(6,7 - dichloro - 2 - cyclopentyl - 2,3 - dihydro - 2 - methyl - 1 - oxo - 1H - inden - 5 - yl)oxy]butanoyl}imidazole used in Example 11B and conducting the reaction as described in Example 11B, there is obtained 1 - carboxy - 1 - methylethyl (+) 4 - [(2 - butyl - 6,7 - dichloro - 2 - cyclopentyl - 2,3 - dihydro - 1 - oxo - 1H - inden - 5 - yl)oxy]butanoate.

Example 13

1-Carboxy-1-methylethyl (−) 4-[(2-butyl-6,7-dichloro-2-cyclopentyl-2,3-dihydro-1-oxo-1H-inden-5-yl)oxy]-butanoate

Step A: 1-{(−) 4-[(2-Butyl-6,7-dichloro-2-cyclopentyl-2,3-dihydro-1-oxo-1H-inden-5-yl)oxy]butanoyl}-imidazole

By substituting an equimolar amount of (−) 4 - [(2 - butyl - 6,7 - dichloro - 2 - cyclopentyl - 2,3 - dihydro - 1 - oxo - 1H - inden - 5 - yl)oxy]butanoic acid for the (+) 4 - [(6,7 - dichloro - 2 - cyclopentyl - 2,3 - dihydro - 2 - methyl - 1 - oxo - 1H - inden - 5 - yl)oxy]butanoic acid used in Example 11A and conducting the reaction as described in Example 11A there is obtained 1 - {(−) 4 - [(2-butyl - 6,7 - dichloro - 2 - cyclopentyl - 2,3 - dihydro - 1 - oxo - 1H - inden - 5 - yl)oxy]butanoyl}imidazole.

Step B. 1-Carboxy-1-methylethyl (−) 4-[(2-butyl-6,7-dichloro-2-cyclopentyl-2,3-dihydro-1-oxo-1H-inden-5-yl)oxy]butanoate

By substituting an equimolar amount of 1 - {(−) 4 - [(2 - butyl - 6,7 - dichloro - 2 - cyclopentyl - 2,3 - dihydro - 1 - oxo - 1H - inden - 5 - yl)oxy]butanoyl}imidazole for the 1 - {(+) 4 - [(6,7 - dichloro - 2 - cyclopentyl - 2,3 - dihydro - 2 - methyl - 1 - oxo - 1H - inden - 5 - yl)oxy]butanoyl}imidazole used in Example 11B and conducting the reaction as described in Example 11B, there is obtained 1 - carboxy - 1 - methylethyl (−) 4 - [(2 - butyl - 6,7 - dichloro - 2 - cyclopentyl - 2,3 - dihydro - 1 - oxo - 1H - inden - 5 - yl)oxy]butanoate.

The compounds of formulae I are utilized by formulating them in a composition such as tablet, capsule or elixir for oral administration. Sterile solutions or suspensions can be used for parenteral administration. about 70 µg to 150 mg of a compound or mixture of compounds of formulae I or a physiologically acceptable salt, ester, or amide is compounded with a physiologically acceptable vehicle, carrier, excipient, binder preservative, stabilizer, flavor, etc. in a unit dosage form as called for by accepted pharmaceutical practice. The amount of active substance in the composition is such that dosage in the range indicated is obtained.

Illustrative of the adjuvants which may be incorporated in tablets, capsules and the like are the following: a binder such as gum tragacanth, acacia, corn starch, potato starch, alginic acid and the like; a lubricant such as magnesium stearate; a sweetening agent such as sucrose, lactose, or saccharin; a flavoring agent such as peppermint, oil of wintergreen or cherry. When the dosage unit form is a capsule, it may contain in addition to materials of the above type a liquid carrier such as a fatty oil. Various other materials may be present as coatings or to otherwise enhance the pharmaceutical elegance of the preparation. For instance, tablets may be coated with shellac, sugar or the like. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a conventional vehicle such as water for injection, a naturally occurring vegetable oil like sesame oil, cocunut oil, peanut oil, cottonseed oil, etc., or a synthetic fatty vehicle like ethyl oleate or the like. Buffers, preservatives, antioxidants and the like can be incorporated as required.

The following examples are included to illustrate the preparation of representative dosage forms.

Example 14

Dry-filled capsules containing 25 mg of active ingredient per capsule

| | Per capsule |
|---|---|
| (+) 4-[6,7-dichloro-2-cyclopentyl-2,3-dihydro-2-methyl-1-oxo-1H-inden-5-yl)oxy]butanoic acid | 25 mg |
| Lactose | 124 mg |
| Magnesium Stearate | 1 mg |
| Capsule (Size No. 1) | 200 mg |

The (+) 4-[6,7-dichloro-2-cyclopentyl-2,3-dihydro-2-methyl-1-oxo-1H-inden-5-yl)oxy]butanoic acid is reduced to a No. 60 powder and then lactose and magnesium stearate are passed through a No. 60 bolting cloth onto the powder and the combined ingredients admixed for 10 minutes and then filled into a No. 1 dry gelatin capsule.

Example 15
Dry-filled capsules containing 25 mg of active ingredient per capsule

| | Per capsule |
|---|---|
| (+) 4-[(2-butyl-6,7-dihloro-2-cyclopentyl-2,3-dihydro-1-oxo-1H-inden-5-yl)oxy]butanoic acid | 25 mg |
| Lactose | 124 mg |
| Magnesium Stearate | 1 mg |
| Capsule (Size No. 1) | 200 mg |

The (+) 4-[(2-butyl-6,7-dichloro-2-cyclopentyl-2,3-dihydro-1-oxo-1H-inden-5-yl)oxy]butanoic acid is reduced to a No. 60 powder and then lactose and magnesium stearate are passed through a No. 60 bolting cloth onto the powder and the combined ingredients admixed for 10 minutes and the filled into a No. 1 dry gelatin capsule.

Example 16
Dry-filled capsules containing 25 mg of active ingredient per capsule

| | Per capsule |
|---|---|
| (−) 4-[(2-butyl-6,7-dichloro-2-cyclopentyl-2,3-dihydro-1-oxo-1H-inden-5-yl)oxy]butanoic acid | 25 mg |
| Lactose | 124 mg |
| Magnesium Stearate | 1 mg |
| Capsule (Size No. 1) | 200 mg |

The (−) 4-[(2-butyl-6,7-dichloro-2-cyclopentyl-2,3-dihydro-1-oxo-1H-inden-5-yl)oxy]butanoic acid is reduced to a No. 60 powder and then lactose and magnesium stearate are passed through a No. 60 bolting cloth onto the powder and the combined ingredients admixed for 10 minutes and then filled into a No. 1 dry gelatin capsule.

Similar dry-filled capsules can be prepared by replacing the active ingredient of the above example by any of the other compounds of this invention.

Example 17
Parenteral solution of the sodium salt of (+) 4 - [(6,7 - dichloro - 2 - cyclopentyl - 2,3 - dihydro - 2 - methyl - 1 - oxo - 1H - inden - 5 - yl)oxy]butanoic acid

100 mg. of (+) 4-[(6,7-dichloro-2-cyclopentyl-2,3-dihydro-2-methyl-1-oxo-1H-inden-5-yl)oxy]butanoic acid is dissolved in 3 ml of 0.1 N-sodium hydrogen carbonate solution. The solution is made up to 10 ml. with water and sterilized by filtration.

Example 18
Parenteral solution of the sodium salt of (−) 4 - [(2 - butyl - 6,7 - dichloro - 2 - cyclopentyl - 2,3 - dihydro - 1 - oxo - 1H - inden - 5 - yl)oxy]butanoic acid

100 mg. of (−) 4-[(2-butyl-6,7-dichloro-2-cyclopentyl-2,3-dihydro-1-oxo-1H-inden-5-yl)oxy]butanoic acid is dissolved in 3 ml. of 0.1 N sodium hydrogen carbonate solution. The solution is diluted with water to a volume of 40 ml. and sterilized by filtration.

Example 19
Parenteral solution of the sodium salt of 1 - carboxy - 1 - methylethyl (+) 4 - [(6,7 - dichloro - 2 - cyclopentyl - 2,3 - dihydro - 2 - methyl - 1 - oxo - 1H - inden - 5 - yl)oxy]butanoate

100 mg. of 1-carboxy-1-methyl (+) 4 - [(6,7 - dichloro - 2 - cyclopentyl - 2,3 - dihydro - 2 - methyl - 1 - oxo - 1H - inden - 5 - yl)oxy]butanoate is dissolved in 3 ml. of 0.1 N sodium hydrogen carbonate solution. The solution is diluted with water to a volume of 40 ml. and sterilized by filtration.

Example 20
Parenteral solution of the sodium salt of 1 - carboxy - 1 - methylethyl (−) 4 - [(2 - butyl - 6,7 - dichloro - 2 - cyclopentyl - 2,3 - dihydro - 1 - oxo - 1H - inden - 5 - yl)oxy]butanoate

100 mg. of 1 - carboxy - 1 - methylethyl (−) 4 - [(2 - butyl - 6,7 - dichloro - 2 - cyclopentyl - 2,3 - dihydro - 1 - oxo - 1H - inden - 5 - yl)oxy]butanoate is dissolved in 3 ml. of 0.1 N sodium hydrogen carbonate. The solution is diluted with water and sterilized by filtration.

Example 21

Parenteral solution of the sodium salt of 1 - carboxy - 1 - methylethyl (+) 4 - [(2-butyl - 6,7 - dichloro - 2 - cyclopentyl - 2,3 - dihydro - 1 - oxo - 1H - inden - 5 - yl)oxy]butanoate

100 mg. of 1-carboxy-1-methylethyl (+) 4 - [(2 - butyl - 6,7 - dichloro - 2 - cyclopentyl - 2,3 - dihydro - 1 - oxo - 1H - inden - 5 - yl)oxy]butanoate is dissolved in 3 ml. of 0.1 N sodium hydrogen carbonate solution. The solution is diluted with water to a volume of 40 ml. and sterilized by filtration.

Example 22

Parenteral solution of the sodium salt of (+) 4 - [(2 - butyl - 6,7 - dichloro - 2 - cyclopentyl - 2,3 - dihydro - 1 - oxo - 1H - inden - 5 - yl)oxy]butanoic acid

100 mg. of (+ 4 - [(2 - butyl - 6,7 - dichloro - 2 - cyclopentyl - 2,3 - dihydro - 1 - oxo - 1H - inden - 5 - yl)oxy]butanoic acid is dissolved in 3 ml. of 0.1 N sodium hydrogen carbonate. The solution is diluted with water to a volume of 10 ml. and sterilized by filtration.

**Claims for the Contracting States BE, CH, DE, FR, GB, IT, LI LU NL SE:**

1. Pharmaceutical composition for treating gray matter edema comprising a compound of the formula

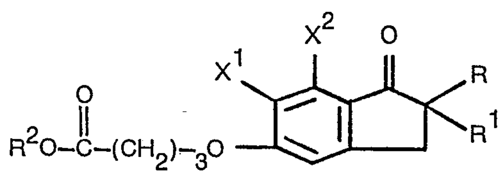

wherein

$X^1$ and $X^2$ are halo;

R is lower alkyl of from 1 to 6 carbon atoms;

$R^1$ is hydrogen, lower alkyl of 1 to 4 carbon atoms, cycloalkyl of 3 to 6 carbon atoms, lowercycloalkylloweralkyl of 4 to 7 carbon atoms and phenyl;

$R^2$ is hydrogen or lower alkyl of 1 to 6 carbon atoms, and carboxy lower alkyl 2 to 6 carbon atoms; and the pharmaceutically acceptable salts thereof;

with the exception of 4-[(6,7-dichloro-2-cyclopentyl-2,3-dihydro-2-methyl-1-oxo-1H-inden-5-yl)oxy]butanoic acid.

2. A composition according to Claim 1 wherein $X^1$ and $X^2$ are chloro.

3. A composition according to Claim 2 wherein $R^1$ is cyclopentyl.

4. A composition according to Claim 3 wherein $R^2$ is H.

5. Pharmaceutical composition for treating gray matter edema comprising 4 - [(6,7 - dichloro - 2 - cyclopentyl - 2,3 - dihydro - 2 - methyl - 1 - oxo - 1H - inden - 5 - yl)oxy]butanoic acid, lactose and magnesium stearate.

6. A composition according to Claim 4 wherein R is ethyl, that is, 4 - [(6,7 - dichloro - 2 - cyclopentyl - 2 - ethyl - 2,3 - dihydro - 1 - oxo - 1H - inden - 5 - yl)oxy]butanoic acid.

7. A composition according to Claim 4 wherein R is propyl, that is, 4 - [(6,7 - dichloro - 2 - cyclopentyl - 2,3 - dihydro - 1 - oxo - 2 - propyl - 1H - inden - 5 - yl)oxy]butanoic acid.

8. A composition according to Claim 4 wherein R is butyl, that is, 4 - [(2 - butyl - 6,7 - dichloro - 2 - cyclopentyl - 2,3 - dihydro - 1 - oxo - 1H - inden - 5 - yl)oxy]butanoic acid.

9. A composition according to Claim 3 wherein R is methyl and $R^2$ is 1 - carboxy - 1 - methylethyl, that is, 1 - carboxy - 1 - methylethyl (+) 4 - [(6,7 - dichloro - 2 - cyclopentyl - 2,3 - dihydro - 2 - methyl - 1 - oxo - 1H - inden - 5 - yl)oxy]butanoate.

10. A composition according to Claim 3 wherein R is butyl and $R^2$ is 1 - carboxy - 1 - methylethyl, that is, 1 - carboxy - 1 - methylethyl (+) 4 - [(2 - butyl - 6,7 - dichloro - 2 - cyclopentyl - 2,3 - dihydro - 1 - oxo - 1H - inden - 5 - yl)oxy]butanoate.

**Claims for the Contracting State AT:**

1. A process for preparing a pharmaceutical composition for treating gray matter edema which comprises compounding a compound of the formula

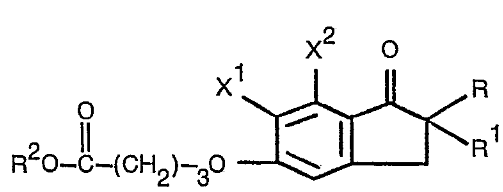

wherein

$X^1$ and $X^2$ are halo;

R is lower alkyl of from 1 to 6 carbon atoms;

$R^1$ is hydrogen, lower alkyl of 1 to 4 carbon atoms, cycloalkyl of 3 to 6 carbon atoms, lowercycloalkylloweralkyl of 4 to 7 carbon atoms and phenyl;

$R^2$ is hydrogen or lower alkyl of 1 to 6 carbon atoms, and carboxy lower alkyl of 2 to 6 carbon atoms; and the pharmaceutically acceptable salts thereof;

with the exception of 4 - [(6,7 - dichloro - 2 - cyclopentyl - 2,3 - dihydro - 2 - methyl - 1 - oxo - 1H - inden - 5 - yl)oxy]butanoic acid; with a physiologically acceptable carrier.

2. A process according to Claim 1, wherein $X^1$ and $X^2$ are chloro.

3. A process according to Claim 2, wherein $R^1$ is cyclopentyl.

4. A process according to Claim 3, wherein $R^2$ is H.

5. A process for preparing a pharmaceutical composition for treating gray matter edema which comprises compounding 4 - [(6,7 - dichloro - 2 - cyclopentyl - 2,3 - dihydro - 2 - methyl - 1 - oxo - 1H - inden - 5 - yl)oxy]butanoic acid with lactose and magnesium stearate.

6. A process according to Claim 4 wherein R is ethyl, that is, 4 - [(6,7 - dichloro - 2 - cyclopentyl - 2 - ethyl - 2,3 - dihydro - 1 - oxo - 1H - inden-5-yl)oxy]butanoic acid.

7. A process according to Claim 4 wherein R is propyl, that is 4 - [(6,7 - dichloro - 2 - cyclopentyl - 2,3 - dihydro - 1 - oxo - 2 - propyl - 1H - inden - 5 - yl)oxy]butanoic acid.

8. A process according to Claim 4 wherein R is butyl, that is, 4 - [(2 - butyl - 6,7 - dichloro - 2 - cyclopentyl - 2,3 - dihydro - 1 - oxo - 1H - inden - 5 - yl)oxy]butanoic acid.

9. A process according to Claim 3 wherein R is methyl and $R^2$ is 1 - carboxy - 1 - methylethyl, that is, 1 - carboxy - 1 - methylethyl (+) 4 - [(6,7 - dichloro - 2 - cyclopentyl - 2,3 - dihydro - 2 - methyl - 1 - oxo - 1H - inden - 5 - yl)oxy]butanoate.

10. A process according to Claim 3 wherein R is butyl and $R^2$ is 1 - carboxy - 1 - methylethyl, that is, 1 - carboxy - 1 - methylethyl (+) 4 - [(2 - butyl - 6,7 - dichloro - 2 - cyclopentyl - 2,3 - dihydro - 1 - oxo - 1H - inden - 5 - yl)oxy]butanoate.

**Patentansprüche für die Vertragsstaaten BE, CH, DE, FR, GB, IT, LI, LU, NL, SE:**

1. Pharmazeutische Zusammensetzung zur Behandlung des Ödems der gruen Substanz, enthaltend eine Verbindung der Formel

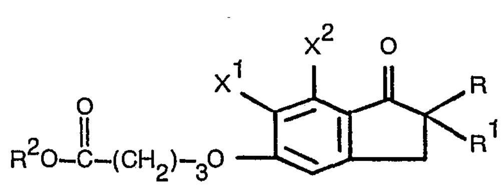

worin

$X^1$ und $X^2$ Hlaogen sind;

R Niedrigalkyl mit 1 bis 6 Kohlenstoffatomen ist;

$R^1$ Wasserstoff, Niedrigalkyl mit 1 bis 4 Kohlenstoffatomen, Cycloalkyl mit 3 bis 6 Kohlenstoffatomen, Niedrigcycloalkylniedrigalkyl mit 4 bis 7 Kohlenstoffatomen oder Phenyl ist;

$R^2$ Wasserstoff oder Niedrigalkyl mit 1 bis 6 Kohlenstoffatomen oder Carboxyniedrigalkyl mit 2 bis 6 Kohlenstoffatomen ist;

oder ein pharmazeutisch annehmbares Salz davon; mit der Ausnahme von 4 - [(6,7 - Dichlor - 2 - cyclopentyl - 2,3 - dihydro - 2 - methyl - 1 - oxo - 1H - inden - 5 - yl)oxy]butansäure.

2. Eine Zusammensetzung nach Anspruch 1, worin $X^1$ und $X^2$ Chlor sind.

3. Eine Zusammensetzung nach Anspruch 2, worin $R^1$ Cyclopentyl ist.

4. Eine Zusammensetzung nach Anspruch 3, worin $R^2$ H ist.

5. Pharmazeutisch Zusammensetzung zur Behandlung von Ödem der grauen Substanz, enthaltend 4 - [(6,7 - Dichlor - 2 - cyclopentyl - 2,3 - dihydro - 2 - methyl - 1 - oxo - 1H - inden - 5 - yl)oxy] - butansäure, Lactose und Magnesiumstearat.

6. Eine Zusammensetzung nach Anspruch 4, worin R Ethyl ist, d.h. worin die Verbindung 4 - [(6,7 - Dichlor - 2 - cyclopentyl - 2 - ethyl - 2,3 - dihydro - 1 - oxo - 1H - inden - 5 - yl)oxy] - butansäure ist.

7. Eine Zusammensetzung nach Anspruch 4, worin R Propyl ist, d.h. worin die Verbindung 4 - [(6,7 - Dichlor - 2 - cyclopentyl - 2,3 - dihydro - 1 - oxo - 2 - propyl - 1H - inden - 5 - yl)oxy] - butansäure ist.

8. Eine Zusammensetzung nach Anspruch 4, worin R Butyl ist, d.h. worin die Verbindung 4 - [(2 - Butyl - 6,7 - dichlor - 2 - cyclopentyl - 2,3 - dihydro - 1 - oxo - 1H - inden - 5 - yl)oxy] - butansäure ist.

9. Eine Zusammensetzung nach Anspruch 3, worin R Methyl ist und $R^2$ 1 - Carboxy - 1 - methylethyl ist, d.h. worin die Verbindung 1 - Carboxy - 1 - methylethyl - (+)4 - [(6,7 - dichlor - 2 - cyclopentyl - 2,3 - dihydro - 2 - methyl - 1 - oxo - 1H - inden - 5 - yl)oxy]butanoat ist.

10. Eine Zusammensetzung nach Anspruch 3, worin R Butyl und $R^2$ 1 - Carboxy - 1 - methylethyl ist,

d.h. worin die Verbindung 1 - Carboxy - 1 - methylethyl - (+)4 - [(2-butyl - 6,7 - dichlor - 2 - cyclopentyl - 2,3 - dihydro - 1 - oxo - 1H - inden - 5 - yl)oxy]butanoat ist.

**Patentansprüche für den Vertragsstaat AT:**

1. Ein Verfahren zur Herstellung einer pharmazeutischen Zusammensetzung für die behandlung des Ödems der grauen Substanz, umfassend das Vermischen einer Verbindung der Formel

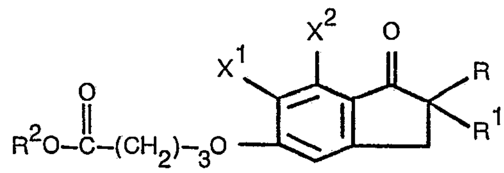

worin
$X^1$ und $X^2$ Halogen sind;
R Niedrigalkyl mit 1 bis 6 Kohlenstoffatomen ist;
$R^1$ Wasserstoff, Niedrigalkyl mit 1 bis 4 Kohlenstoffatomen, Cycloalkyl mit 3 bis 6 Kohlenstoffatomen, Niedrigcycloalkylniedrigalkyl mit 4 bis 7 Kohlenstoffatomen oder Phenyl ist;
$R^2$ Wasserstoff oder Niedrigalkyl mit 1 bis 6 Kohlenstoffatomen oder Carboxyniedrigalkyl mit 2 bis 6 Kohlenstoffatomen ist;
Oder eines pharmazeutisch annehmbaren Salzes davon; mit der Ausnahme von 4 - [(6,7 - Dichlor - 2 - cyclopentyl - 2,3 - dihydro - 2 - methyl - 1 - oxo - 1H - inden - 5 - yl)oxy]butansäure, mit einem physiologisch annehmbaren Träger.

2. Ein Verfahren nach Anspruch 1, worin $X^1$ und $X^2$ Chlor sind.

3. Ein Verfahren nach Anspruch 2, worin $R^1$ Cyclopentyl ist.

4. Ein Verfahren nach Anspruch 3, worin $R^2$ H ist.

5. Ein Verfahren zur Herstellung einer pharmazeutischen Zusammensetzung für die Behandlung von Ödem der grauen Substanz, umfassend das Vermischen von 4 - [(6,7 - Dichlor - 2 - cyclopentyl - 2,3 - dihydro - 2 - methyl - 1 - oxo - 1H - inden - 5 - yl)oxy] - butansäure mit Lactose und Magnesiumstearat.

6. Ein Verfahren nach Anspruch 4, worin R Ethyl ist, d.h. worin die Verbindung 4 - [(6,7 - Dichlor - 2 - cyclopentyl - 2 - ethyl - 2,3 - dihydro - 1 - oxo - 1H - inden - 5 - yl)oxy] - butansäure ist.

7. Ein Verfahren nach Anspruch 4, worin R Propyl ist, d.h. worin die Verbindung 4 - [(6,7 - Dichlor - 2 - cyclopentyl - 2,3 - dihydro - 1 - oxo - 2 - propyl - 1H - inden - 5 - yl)oxy] - butansäure ist.

8. Ein Verfahren nach Anspruch 4, worin R Butyl ist, d.h. worin die Verbindung 4 - [(2 - Butyl - 6,7 - dichlor - 2 - cyclopentyl - 2,3 - dihydro - 1 - oxo - 1H - inden - 5 - yl)oxy] - butansäure ist.

9. Ein Verfahren nach Anspruch 3, worin R Methyl ist und $R^2$ 1 - Carboxy - 1 - methylethyl ist, d.h. worin die Verbindung 1 - Carboxy - 1 - methylethyl - (+)4 - [(6,7 - dichlor - 2 - cyclopentyl - 2,3 - dihydro - 2 - methyl - 1 - oxo - 1H - inden - 5 - yl)oxy]butanoat ist.

10. Ein Verfahren nach Anspruch 3, worin R Butyl und $R^2$ 1 - Carboxy - 1 - methylethyl ist, d.h. worin die Verbindung 1 - Carboxy - 1 - methylethyl - (+)4 - [(2 - butyl - 6,7 - dichlor - 2 - cyclopentyl - 2,3 - dihydro - 1 - oxo - 1H - inden - 5 - yl)oxy]butanoat ist.

**Revendications pour les Etats Contractants BE, CH, DE, FR, GB, IT, LI, LU, NL, SE:**

1. Composition pharmaceutique pour le traitement de l'oedème de la substance grise, comprenant un composé de formule:

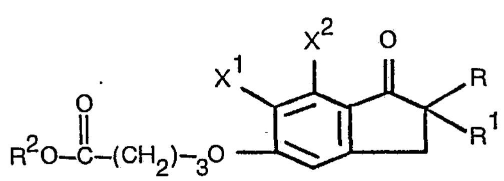

dans laquelle
$X^1$ et $X^2$ sont un halogéno;
R est un alkyl inférieur de 1 à 6 atomes de carbone;
$R^1$ est un hydrogène, un alkyle inférieur de 1 à 4 atomes de carbone, un cycloalkyle de 3 à 6 atomes de carbone, un cycloalkyl(inférieur)alkyle inférieur de 4 à 7 atomes de carbone ou un phényle;
$R^2$ est un hydrogène ou un alkyl inférieur de 1 à 6 atomes de carbone ou un carboxyalkyle inférieur de 2 à 6 atomes de carbone; et leurs sels acceptables en pharmacie;
à l'exception de l'acide 4 - [(6,7 - dichloro - 2 - cyclopentyl - 2,3 - dihydro - 2 - méthyl - 1 - oxo - 1H - indène - 5 - yl)oxy]butanoïque.

2. Une composition selon la revendication 1, dans laquelle $X^1$ et $X^2$ sont un chloro.

3. Une composition selon la revendication 2 dans laquelle $R^1$ est un cyclopentyl.

4. Une composition selon la revendication 3 dans laquelle $R^2$ est H.

5. Une composition pharmaceutique pour le traitement de l'oedème de la substance grise, comprenant l'acide 4 - [(6,7 - dichloro - 2 - cyclopentyl - 2,3 - dihydro - 2 - méthyl - 1 - oxo - 1H - indène - 5 - yl)oxy]butanoïque, du lactose et du stéarate de magnésium.

6. Une composition selon la revendication 4, dans laquelle R est un éthyle, c'est-à-dire l'acide 4 - [(6,7 - dichloro - 2 - cyclopentyl - 2 - éthyl - 2,3 - dihydro - 1 - oxo - 1H - indène - 5 - yl)oxy]butanoïque.

7. Une composition selon la revendication 4, dans laquelle R est un propyle, c'est-à-dire l'acide 4 - [(6,7 - dichloro - 2 - cyclopentyl - 2,3 - dihydro - 1 - oxo - 2 - propyl - 1H - indène - 5 - yl)oxybutanoïque.

8. Une composition selon la revendication 4 dans laquelle R est un butyle, c'est-à-dire l'acide 4 - [(2 - butyl - 6,7 - dichloro - 2 - cyclopentyl - 2,3 - dihydro - 1 - oxo - 1H - indène - 5 - yl)oxybutanoïque.

9. Une composition selon la revendication 3 dans laquelle R est un méthyle et $R^2$ est un 1 - carboxy - 1 - méthyléthyle, c'est-à-dire le (+)4 - [(6,7 - dichloro - 2 - cyclopentyl - 2,3 - dihydro - 2 - méthyl - 1 - oxo - 1H - indène - 5 - yl)oxybutanoate de 1 - carboxy - 1 - méthyléthyle.

10. Une composition selon la revendication 3 dans laquelle R est un butyle et $R^2$ est un 1 - carboxy - 1 - méthyléthyle, c'est-à-dire le (+)4 - [(2 - butyl - 6,7 - dichloro - 2 - cyclopentyl - 2,3 - dihydro - 1 - oxo - 1H - indène - 5 - yl)oxy]butanoate de 1 - carboxy - 1 - méthyléthyle.

**Revendications pour l'Etat Contractant AT:**

1. Un procédé pour la préparation d'une composition pharmaceutique pour le traitement de l'oedème de la substance grise qui comprend la combinaison d'un composé de formule:

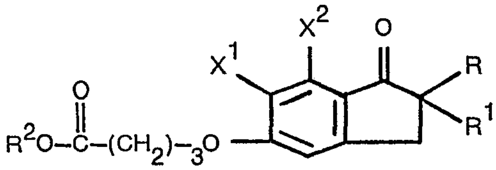

dans laquelle

$X^1$ et $X^2$ sont un halogéno;

R est un alkyl inférieur de 1 à 6 atomes de carbone;

$R^1$ est un hydrogène, un alkyle inférieur de 1 à 4 atomes de carbone, un cycloalkyle de 3 à 6 atomes de carbone, un cycloalkyl(inférieur)alkyl inférieur de 4 à 7 atomes de carbone ou un phényle;

$R^2$ est un hydrogène ou un alkyle inférieur de 1 à 6 atomes de carbone, ou un carboxyalkyle inférieur de 2 à 6 atomes de carbone; et leurs sels acceptables en pharmacie;

à l'exception de l'acide 4 - [(6,7 - dichloro - 2 - cyclopentyl - 2,3 - dihydro - 2 - méthyl - 1 - oxo - 1H - indène - 5 - yl)oxy]butanoïque; avec un support physiologiquement acceptable.

2. Un procédé selon la revendication 1 dans lequel $X^1$ et $X^2$ sont un chloro.

3. Un procédé selon la revendication 2 dans lequel $R^1$ est un cyclopentyle.

4. Un procédé selon la revendication 3 dans lequel $R^2$ est H.

5. Un procédé pour préparer une composition pharmaceutique pour le traitement de l'oedème de la substance grise qui comprend la combinaison de l'acide 4 - [(6,7 - dichloro - 2 - cyclopentyl - 2,3 - dihydro - 2 - méthyl - 1 - oxo - 1H - indène - 5 - yl)oxy]butanoïque avec du lactose et du stéarate de magnésium.

6. Un procede selon la revendication 4 dans lequel R est un éthyle, c'est-à-dire l'acide 4 - [(6,7 - dichloro - 2 - cyclopentyl - 2 - éthyl - 2,3 - dihydro - 1 - oxo - 1H - indène - 5 - yl)oxy]butanoïque.

7. Un procédé selon la revendication 4, dans lequel R est un propyle, c'est-à-dire l'acide 4 - [(6,7 - dichloro - 2 - cyclopentyl - 2,3 - dihydro - 1 - oxo - 2 - propyl - 1H - indène - 5 - yl)oxy]butanoïque.

8. Un procédé selon la revendication 4, dans lequel R est un butyle, c'est-à-dire l'acide 4 - [(2 - butyl - 6,7 - dichloro - 2 - cyclopentyl - 2,3 - dihydro - 1 - oxo - 1H - indène - 5 - yl)oxy]butanoïque.

9. Un procédé selon la revendication 3, dans lequel R est un méthyle et $R^2$ est un 1 - carboxy - 1 - méhyléthyle c'est-à-dire le (+)4 - [(6,7 - dichloro - 2 - cyclopentyl - 2,3 - dihydro - 2 - méthyl - 1 - oxo - 1H - indène - 5 - yl)oxy]butanoate de 1 - carboxy - 1 - méthyléthyle.

10. Un procédé selon la revendication 3 dans lequel R est un butyle et $R^2$ est un 1 - carboxy - 1 - méthyléthyle, c'est-à-dire le (+)4 - [(2 - butyl - 6,7 - dichloro - 2 - cyclopentyl - 2,3 - dihydro - 1 - oxo - 1H - indène - 5 - yl)oxy]butanoate de 1 - carboxy - 1 - méthyléthyle.